# EUROPEAN PATENT APPLICATION

(11) **EP 2 668 890 A1**
(43) Date of publication of application: **04.12.2013**
(21) Application number: 12739693.5
(22) Date of filing: 30.01.2012
(51) Int. Cl.: A61B 1/00, A61B 19/02

(54) **ENDOSCOPE SYSTEM CARRYING BAY AND ENDOSCOPE SYSTEM WORK STATION**

(30) Priority: 28.01.2011 CN 201110035054
(71) Applicant: Olympus (Beijing) Sales & Service Co., Ltd., Beijing 100027 (CN)
(72) Inventor: XU, Junwei, Shanghai 200031 (CN); HUANG, Xin, Shanghai 200031 (CN)
(74) Representative: Grünecker, Kinkeldey, Stockmair & Schwanhäusser
(86) International application number: PCT/CN2012/000135
(87) International publication number: WO 2012/100663

(57) **Abstract**

The present invention provides an endoscope system carrying portion which has good integrity and can hide the wires, and a workstation capable of integrating the endoscope system carrying portion. The endoscope system carrying portion is provided with a trolley storage portion for accommodating a trolley which is used for carrying medical equipment including at least an endoscope driving apparatus. By means of accommodating the trolley in the trolley storage portion of the endoscope system carrying portion, the various wires on the trolley which were originally messy are hidden so as to increase the integrity of the entire endoscope system carrying portion. In order to provide a convenient and comfortable operating environment to the medical personnel by disposing a little number of apparatus in the surgery without the exposed trolley, and enhance the quality and appearance of the hospital. At the same time to ease the fear among patients.

## Description

### Cross Reference of Related Application

The present application depends on and claims the right of priority of the Chinese patent application No. 201110035054.8 filed on January 28, 2011. All the contents of the Chinese patent application No. 201110035054.8 are thereby incorporated into the present application.

### Field of the Invention

The present invention relates to the field of medical facilities, particularly to an endoscope system carrying portion for integrating a trolley, and a workstation.

### Description of the Related Art

In the medical field, when it comes to medical examination and treatment, sometimes an endoscope is needed to help with the diagnosis and treatment. To be specific, an endoscope is inserted into the body cavity of a person, and then an optical assembly at the top of the endoscope examines the inside of the body cavity. In the past, the equipment for diagnosis and treatment, which was connected to the endoscope, was carried by a trolley. Although such a design makes it easier for the doctors and nurses to move the equipment as a whole, there is usually a strict demand for the hygiene of the endoscope as it has to be inserted into the human body. The patients always hope that the treatment is carried out in a place that is safe and sanitary, and the doctors also want to prevent cross contamination to thereby ensure the safety of the patients.

As stated above, at present the endoscope is usually carried by a trolley. During the diagnosis and treatment, in addition to the endoscope, some auxiliary devices such as the monitor are also used. Although the auxiliary devices can be mounted to the trolley which carries the endoscope or to another trolley, this makes an unfavorable impact on the entire arrangement of the diagnosis and treatment area. No matter how many trolleys are used, the connection among the devices requires a great number of connecting wires and most of the wires have to be arranged on the floor, which makes the wiring on the floor a mess. The more trolleys are used, the messier the wiring becomes. Even if only one trolley is used and integrated wiring is employed, there will still be a great number of connecting wires exposed on the surface of the trolley. The messy wiring and the exposed wires not only make the patients feel anxious but also cause inconvenience to the operation of the doctors or nurses.

JP2001-120492, for example, discloses an endoscope apparatus in which a cantilever monitor bracket is provided at an upper end of a trolley. The monitor bracket is rotatable within a certain angle, which allows the doctor to adjust the position and the angle of the monitor as he or she likes. Furthermore, the lower part of the trolley is provided with a chair, so the doctor can sit and carry out the examination if the examination takes a long period of time.

According to the above description of JP2001-120492, the trolley is provided with a monitor bracket, but the monitor bracket hinders the operation of the doctor to some extent. Moreover, providing the trolley with a monitor makes it inconvenient for the doctors and nurses to observe the endoscopic image. Mounting a number of peripheral machines for diagnosis and treatment to one trolley makes it difficult to detach them from the trolley and influences the heat dissipation of the machines.

In addition, JP2010-5256 also discloses a trolley for an endoscope. The upper part of the trolley is provided with a monitor and an endoscope bracket, and a cover is provided at the outside of the monitor and the endoscope bracket. Several slide fasteners are provided on the front part of the cover to form windows that can be opened or closed according to the actual need. The cover provides better protection to the devices carried on the trolley, prevents the devices from being polluted by the environment, and can accommodate various wires. Furthermore, some components such as the endoscope bracket can be removed from the trolley, which makes it more convenient to adjust the arrangement of the trolley.

However, the design of the trolley disclosed by JP2010-5256 has some defects. For example, the slide fasteners may cause trouble to the opening of the windows; the slide fasteners get damaged easily; and the cover affects the heat dissipation and deteriorates the heat dissipation performance of the whole equipment. Moreover, the integrity of the trolley and the observation device in the diagnosis and treatment area is poor and the wires are not properly integrated.

Further, JPH06-245895, for example, discloses a storage trolley for storing an endoscope system. In order to prevent the endoscope from being polluted, the trolley is designed to have the structure of a box. The front portion and the rear portion of the trolley are provided with openings so that it is convenient to connect the lines. The shielding function of the housing in such a design protects the endoscope, but the peripheral devices for diagnosis and treatment are all exposed to the outside of the trolley and as a result the lines are in a mess.

As described above, in the prior art the devices are mounted to the trolley or bracket and are exposed to the environment. Furthermore, in the prior art, the power supply lines and the signal lines of the devices interlace with each other and thus cause disorder. Even if a monitor is provided on the trolley, the position of the monitor cannot be greatly adjusted as the monitor is mounted to the trolley. Using an independent floor bracket for the monitor also causes inconvenience to the operation of the doctor and makes the whole environment look disorderly.

### Summary of the Invention

The present invention comes into being in view of the above-mentioned defects. One object of the present invention is to provide an endoscope system carrying portion that has good integrity and hides the wires from sight. Another object of the present invention is to provide a workstation capable of integrating the endoscope system carrying portion.

A technical solution of the present invention provides an endoscope system carrying portion which is provided with a trolley storage portion for accommodating a trolley. The trolley is used for carrying medical equipment including at least a driving apparatus for the endoscope.

By means of accommodating the trolley in the trolley storage portion of the endoscope system carrying portion, the various wires on the trolley which were originally messy are hidden so as to increase the integrity of the entire endoscope system carrying portion. In order to provide a convenient and comfortable operating environment to the medical personnel by disposing a little number of apparatus in the surgery without the exposed trolley, and enhance the quality and appearance of the hospital. At the same time to ease the fear among patients.

Another technical solution of the present invention provides an endoscope system carrying portion, with the trolley storage portion being a recessed portion which opens rearward on the endoscope system carrying portion. On the front portion of the endoscope system carrying portion, an opening which communicates with the trolley storage portion is provided in a position corresponding to the trolley storage portion so that the medical personnel can operate the medical equipment carried on the trolley via the opening. A door capable of closing the opening is provided at the opening.

By opening the door, the medical personnel can directly operate the equipment on the trolley, thereby improving the operability of the endoscope system carrying portion.

Another technical solution of the present invention provides an endoscope system carrying portion. The door of the endoscope system carrying portion consists of a first door and a second door. The first door is arranged in a position corresponding to frequently-used buttons and/or interfaces of the equipment for diagnosis and treatment carried on the trolley. Preferably, the first door is smaller than the second door.

Thus, when the medical personnel carry out common operations to the equipment on the trolley, they only need to open the first door, which reduces the exposed area of the trolley and eases the uncomfortable feeling of the patient. Only when complicated operations are to be carried out do the medical personnel open both the first and the second doors. When the first door is smaller than the second door, only opening the first door can more effectively reduce the exposed area of the trolley, make the entire equipment look tidy and clean, and ease the uncomfortable feeling of the patient.

The endoscope system carrying portion is further provided with a storing space which has an opening at the front portion of the endoscope system carrying portion. A door for the storage space that can be slidably opened and closed is provided at the opening. In this way, the space for holding apparatuses (e.g. ESG-100, AFU-100) is increased, and the easy opening of the door of the storing space facilitates the operation of the devices within the storing space.

The front portion of the endoscope system carrying portion is further provided with a temporary storing area for a suction duct. The temporary storing area is a rubber cap having a bottom. The rubber cap has a hole that opens toward the front of the endoscope system carrying portion and engages with the head of the suction duct. While the endoscope is being changed, the temporary storing area provides a place for temporarily storing the suction duct.

The endoscope system carrying portion further comprises a suction line for the suction duct to pass through. The inside of the endoscope system carrying portion is provided with a space through which the suction line penetrates from the front side of the endoscope system carrying portion to the rear side of the endoscope system carrying portion. In a position on the suction line that is slightly behind the front port of the suction line, at least a pair of engaging protrusions is provided in at least a group of radially opposite positions. At the same time, in the space for accommodating the suction line, there is an engaging portion capable of engaging with the engaging protrusions to prevent the suction line from being pulled out. The engagement between the engaging portion and the engaging protrusions is effected by rotating the suction line after the suction line is inserted into the space for accommodating the suction line. Such a design makes it easy to detach the suction line (Knob structure is used, the user can readily detach the suction line by simply turning and pulling the suction line, without using any special tools).

Another technical solution of the present invention provides an endoscope system carrying portion which is provided with a wire arrangement box. The rear portion of the wire arrangement box communicates with the trolley storage portion. The wire arrangement box has an opening on the front portion of the endoscope system carrying portion. The wire arrangement box has a door at its opening, and the door for the wire arrangement box is provided with a damping mechanism for releasing and retracting the wires.

Another technical solution of the present invention provides an endoscope system carrying portion in which the damping mechanism for releasing and retracting the wires is provided on the upper portion of the door for the wire arrangement box and is constituted by a plurality of sheet-like rubber bodies. The plurality of sheet-like rubber bodies are arranged transversely on the door for the wire arrangement box. By means of the sheet-like rubber bodies, a damping force is applied to the wires that are released and retracted via the door for the wire arrangement box.

Another technical solution of the present invention provides an endoscope system carrying portion in which a trolley guiding mechanism for guiding the trolley is provided in the recessed portion. The trolley guiding mechanism is provided at the bottom of the recessed portion and has a shape that matches with the shape of the wheels of the trolley.

Another technical solution of the present invention provides an endoscope system carrying portion which further comprises at least one of the following devices: a sub-monitor provided on the upper portion of the endoscope system carrying portion, a pedal provided on the lower portion of the endoscope system carrying portion, a white balance cap provided on the side portion of the endoscope system carrying portion, a suction bottle provided on the rear portion of the endoscope system carrying portion, and adjusting feet provided on the bottom of the endoscope system carrying portion.

Another technical solution of the present invention provides an endoscope system carrying portion which is further provided with a flat first end face. At least one joining opening is provided at the first end face, and a drawer or cover plate is provided at the joining opening.

Another technical solution of the present invention provides an endoscope system carrying portion which is an elongated structure. The trolley storage portion is provided near an end portion of the endoscope system carrying portion. Near the other end portion of the endoscope system carrying portion, there is a bracket for supporting a main monitor.

Thus, it is possible to provide an elongated endoscope system carrying portion which has a main monitor. By providing the trolley storage portion on one end of the endoscope system carrying portion and providing the main monitor bracket on the other end of the endoscope system carrying portion, it is possible to provide an endoscope system carrying portion that has an integrated structure and has the characteristics of workstation.

The main monitor bracket comprises a main strut rod and a universal adjusting mechanism that is disposed on the upper end of the main strut rod and consists of a plurality of sub strut rods. The universal adjusting mechanism is provided with a damper for retaining the universal adjusting mechanism in an adjusted position. The main strut rod is provided on an end portion of the endoscope system carrying portion in a forward/backward direction, and the sub strut rods extend in the forward/backward direction in a manner of transversely striding over the endoscope system carrying portion. If the main strut rod is arranged on an end portion of the endoscope system carrying portion in a width direction and the sub strut rods which are a cantilever portion extend toward the outer side of the endoscope system carrying portion, a weighted structure is needed. Therefore, by providing the main strut rod on an end portion of the endoscope system carrying portion in a forward/backward direction and extending the sub strut rods in the forward/backward direction, the sub strut rods which are a cantilever portion can extend above the endoscope system carrying portion in the width direction of the endoscope system carrying portion, and the center of gravity of the entire system is relatively stable, and the weighted structure in the above situation can be cancelled due to the weight of the main strut rod. Furthermore, the design in the present technical solution makes the installation more convenient and increases the available space on the front part the operating desk.

Another technical solution of the present invention provides an endoscope system carrying portion in which the main monitor bracket comprises a main strut rod and a universal adjusting mechanism that is disposed on the upper end of the main strut rod and consists of a plurality of sub strut rods. The universal adjusting mechanism is provided with a damper for retaining the universal adjusting mechanism in an adjusted position. Furthermore, the endoscope system carrying portion is provided with a weight for preventing the endoscope system carrying portion from overturning. According to this structure, a main monitor bracket structure with a specific structure is provided. The main monitor bracket having the above-described structure makes it possible for the main monitor to have good universal adjustment performance and makes it convenient for the medical personnel to perform the observation and the operation at the same time.

Another technical solution of the present invention provides an endoscope system workstation which comprises an operation portion and an endoscope system carrying portion. The endoscope system carrying portion and the operation portion can be joined together by a joining mechanism. There is no limitation on the joining manner; that is, the endoscope system carrying portion and the operation portion can be joined together in any manner. Or, said another technical solution of the present invention provides an endoscope system workstation which comprises an operation portion and the endoscope system carrying portion, wherein joining halves of the endoscope system carrying portion are provided at the joining openings and protrude from the endoscope system carrying portion or are recessed into the inside of the joining openings; the operation portion has a second end face that fits with the first end face of the endoscope system carrying portion; and the second end face is provided with joining halves of the operation portion that fit with the joining halves of the endoscope system carrying portion.

Another technical solution of the present invention provides an endoscope system workstation in which the operation portion is provided with a main monitor bracket. The main monitor bracket is disposed at a middle portion of the operation portion and includes a main strut rod and a universal adjusting mechanism that is disposed on the upper end of the main strut rod. The universal adjusting mechanism consists of a plurality of sub strut rods and is provided with a damper for retaining the universal adjusting mechanism in an adjusted position. The lower portion of the operation portion is provided with a weight for preventing the operation portion from overturning.

The operation portion is provided with a main monitor bracket which comprises a main strut rod and a universal adjusting mechanism that is disposed on the upper end of the main strut rod and consists of a plurality of sub strut rods. The universal adjusting mechanism is provided with a damper for retaining the universal adjusting mechanism in an adjusted position. The main strut rod is arranged on an end portion of the operation portion in a forward/backward direction, and the sub strut rods extend in the forward/backward direction in a manner of transversely striding over the operation portion. If the main strut rod is arranged on an end portion or a middle portion of the operation portion in a forward/backward direction and the sub strut rods which are a cantilever portion extend toward the outer side of the operation portion, a weighted structure is needed in order to ensure the stability of the entire endoscope system workstation. Therefore, by providing the main strut rod on an end portion of the operation portion in a forward/backward direction and extending the sub strut rods in the forward/backward direction, the sub strut rods which are a cantilever portion can extend above the operation portion in the width direction of the operation portion, and the center of gravity of the entire system is relatively stable, and the weighted structure in the above situation can be cancelled due to the weight of the main strut rod. Furthermore, the design in the present technical solution makes the installation of the main monitor bracket more convenient and increases the available space on the front part of the operating desk.

### Brief Description of the Drawings

FIG. 1 is a perspective view showing a state in which the endoscope system carrying portion and the operation portion are joined together according to one embodiment of the present invention.

FIG. 2 is a perspective view of the endoscope system carrying portion, showing a state in which the drawers have been installed.

FIG. 3 shows a state in which the endoscope system carrying portion is provided with a carrying portion joining mechanism.

FIG. 4(a) is a perspective view of the combined endoscope system carrying portion and the operation portion in a state in which the trolley is not yet inserted into the endoscope system carrying portion, when viewed from the rear side.

FIG. 4(b) is a lateral view of the endoscope system carrying portion only, showing a state in which the trolley has been inserted into the endoscope system carrying portion.

FIG. 5 is a schematic view showing the relationship between the trolley and the trolley guiding mechanism.

FIG. 6(a) is a schematic view of the first door, the second door and their surrounding portions, showing a state in which the first and the second doors are shut.

FIG. 6(b) is a schematic view of the first door, the second door and their surrounding portions, showing a state in which the first and the second doors are open.

FIG. 7(a) is a schematic view of the door for the wire arrangement box and its surrounding portions, showing a state in which the door for the wire arrangement box is shut.

FIG. 7(b) is a schematic view of the door for the wire arrangement box and its surrounding portions, showing a state in which the door for the wire arrangement box is open.

FIG. 8 is a schematic view of the door for the wire arrangement box and its surrounding portions, showing a front view of the door for the wire arrangement box when it is open.

FIG. 9 is a perspective view of the endoscope system carrying portion according to another embodiment of the present invention.

FIG. 10 is a schematic view of a variant of the trolley storage portion of the present invention.

FIG. 11 is a schematic view of another variant of the trolley storage portion of the present invention.

FIG. 12 is a schematic view of another variant of the trolley storage portion of the present invention.

FIG. 13 is a schematic view of another variant of the trolley storage portion of the present invention.

FIG. 14 is a perspective view showing a state in which the endoscope system carrying portion and the operation portion are joined together according to another embodiment of the present invention.

FIG. 15 is a schematic structural view showing how the suction line is mounted to the endoscope system carrying portion.

### Detailed Description of the Preferred Embodiments

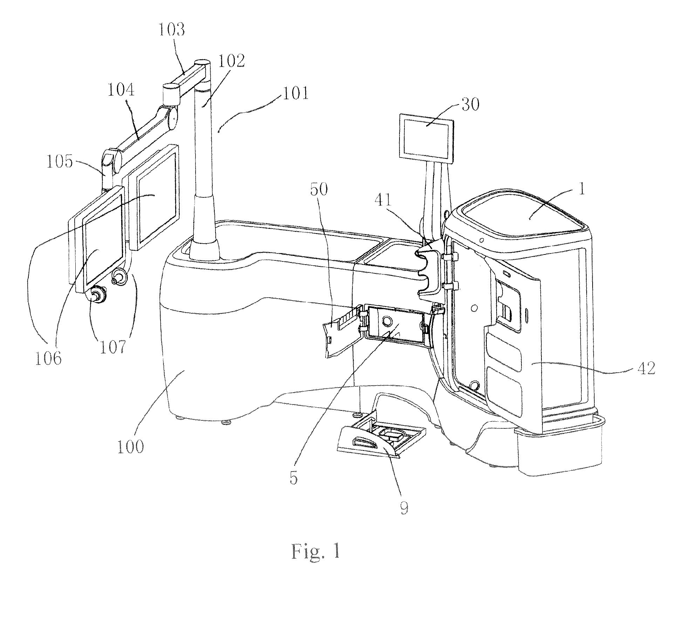

An embodiment of the present invention will be set forth with reference to FIGS. 1-8. Please note that the surface of the workstation that normally faces the medical personnel when the workstation is in normal use is the front side, and the opposite surface of the workstation is the rear side.

First, the main structure of the endoscope system workstation according to the present embodiment will be set forth with reference to FIGS. 1-2.

As shown in FIG. 1, the medical endoscope system workstation (hereinafter referred to as the workstation) of the present embodiment is a detachable structure. The endoscope system workstation comprises an endoscope system carrying portion 1 (hereinafter sometimes referred to as the carrying portion 1) and an operation portion 100. The endoscope system carrying portion 1 and the operation portion 100 are joined together by means of a joining mechanism. The joining mechanism will be described in detail later on.

A main monitor bracket 101 is vertically provided on the operation portion 100 and is used for hanging a plurality of main monitors 106. The main monitor bracket 101 comprises a main strut rod 102 and a plurality of sub strut rods 103-105. A first sub strut rod 103 is connected to an upper end of the main strut rod 102 and laterally extends from the upper end of the main strut rod 102. A second sub strut rod 104 is connected to an outer end of the first sub strut rod 103 via a universal adjusting mechanism having a damper. The second sub strut rod 104 also extends laterally. A third sub strut rod 105 is connected to an outer end of the second sub strut rod 104 via a universal adjusting mechanism having a damper. The third sub strut rod 105 is T-shaped and extends downward from an outer end of the second sub strut rod 104 and then branches leftward and rightward, and two main monitors 106 are mounted to the two outer ends of the left and the right branches of the third sub strut rod 105 in a horizontal direction. Handles 107 that extend from the main monitors 106 are provided at lower sides of the main monitors 106, respectively. The handles 107 can be used to adjust the position and the angle of the main monitors. It is also feasible to provide a universal adjusting mechanism having a damper at the branching position of the third sub strut rod 105 so that it is possible to respectively adjust the two main monitors 106. In the present embodiment, the handles 107 for adjusting the position and the angle of the main monitors are made of a fungi-proofing material and can be replaced regularly to guarantee hygiene.

Furthermore, in the present example, the adjustment of the position and the angle of the main monitors is effected by means of three sub strut rods, but the present invention is not limited to this. It is also feasible to use only two sub strut rods or more than three sub strut rods to adjust the position and the angle of the main monitor. Or, it is also feasible to use stretchable main strut rod and sub strut rods so as to further facilitate the adjustment of the angle and the orientation of the main monitors. Furthermore, the number of the main monitor is not limited to the number in the present embodiment; in other words, there can be only one main monitor or more than two main monitors. That is, the main monitor can be set in any manner according to the need of the doctor.

Furthermore, in the present embodiment, the main monitor bracket 101 is arranged in a corner of the operation portion 100 (when the operation portion 100 is viewed as a whole, the main monitor bracket 101 is located at the middle portion of the operation portion 100), but the present invention is not limited to the present embodiment. In other words, the main monitor bracket 101 can be arranged at any position on the operation portion 100 based on the actual need. In order to keep a balance of the operation portion 100 to prevent it from overturning, in the present embodiment the operation portion 100 is provided with a weight. Thus, even when the operation portion 100 is not connected to the endoscope system carrying portion 1, it can still stand on the ground stably.

Further, as shown in FIG. 1, in the present embodiment, the operation portion 100 has a curvature of a predetermined radian. However, it is not necessary to have such a curvature. The operation portion 100 can be in any desired shape according to the actual situation.

The following relates a description of the endoscope system carrying portion 1. As shown in FIGS. 1-4, the design of the contour line of the carrying portion 1 is similar to that of the operation portion 100, i.e., having a certain radian. The purpose of such a design is to prevent the carrying portion 1 from overturning. However, in other embodiments, according to the actual need, the carrying portion 1 can be designed to have an elongated shape without any radian, or to have a square shape. There is no special limitation on the shape of the carrying portion 1 as long as a recessed portion 2 for accommodating a trolley 8 can be provided at the rear portion of the carrying portion 1 and the operation equipment used by the medial personnel for operating for example an endoscope can be arranged to the front portion of the carrying portion 1. That is, the carrying portion 1 may has any shape.

First, the description of the endoscope system carrying portion 1 begins with the rear side of the carrying portion 1.

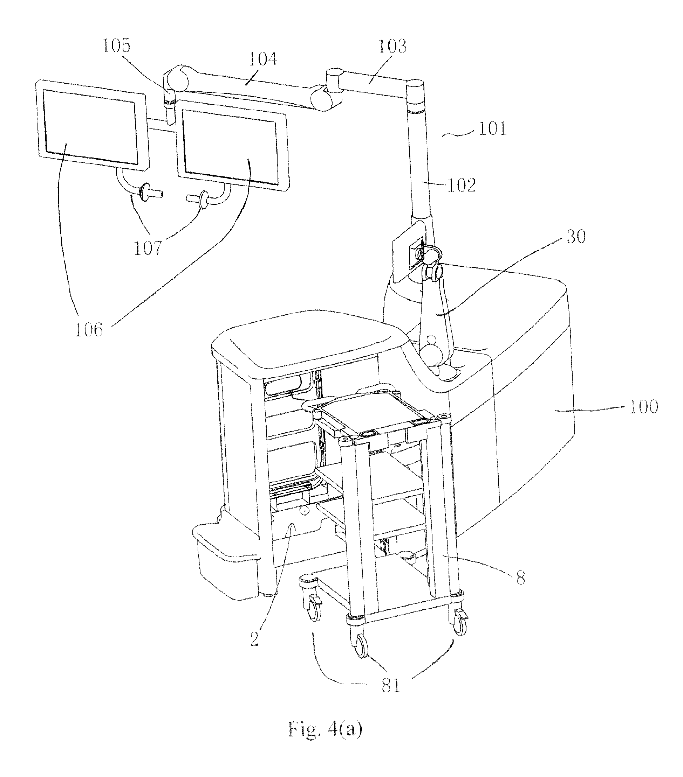

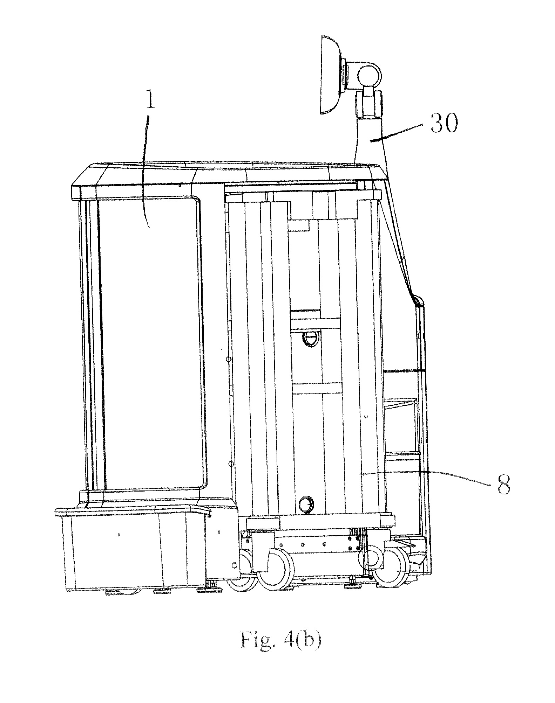

As shown in Fig. 4, the rear portion of the carrying portion 1 is provided with a recessed portion 2 for accommodating the trolley 8. The recessed portion 2 functions as a trolley storage portion. There is no special limitation on the shape of the recessed portion 2; however, from the perspective of practical application, the shape of the recessed portion 2 is preferably to match with the shape of the trolley 8. In the present embodiment, the shape of the recessed portion matches with the shape of e.g. the trolley WM-NPI which is a product of the applicant of the present application and is on the market, i.e., a substantially cubic shape that is recessed inwardly. Furthermore, in order to have a clean and tidy medical environment, preferably, the rear portion of the trolley 8 is substantially aligned with the rear portion of the carrying portion 1 after the trolley 8 is accommodated in the recessed portion 2. Thus, the room for diagnosis and treatment looks cleaner and tidier.

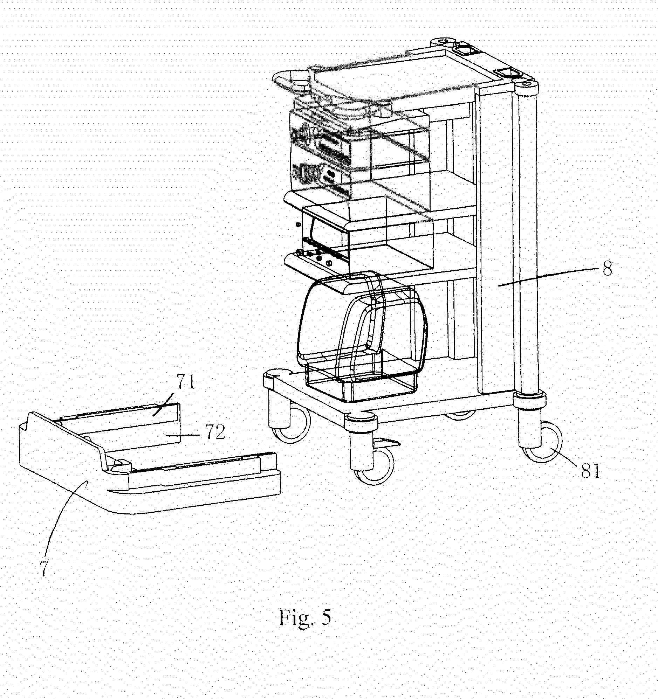

The lower portion of the recessed portion 2 is provided with a guide mechanism 7 which guides wheels 81 of the trolley 8. FIG. 5 is a perspective view of the guide mechanism 7, showing that the guide mechanism 7 comprises a front plate and two side plates. In the present embodiment, the front plate and the side plates form an integral structure. However, the present invention is not limited to this; in other words, the front plate and the side plates can be independent of each other according to the actual need. It is not shown in FIG. 5 that the surface of the front plate is provided with a reversed V-shaped guide projection that guides the wheels 81 of the trolley 8 toward the outer sides. By means of the guide projection, the wheels 81 of the trolley 8 that are pushed into the guide mechanism 7 can be guided toward both sides of the front plate, so the trolley 8 won't leave the guide mechanism 7 easily (in other words, the trolley 8 won't leave the recessed portion 2 easily).

Each of the two side plates is provided with a guide recess 72 which guides the wheels. After the guide mechanism 7 is mounted to the carrying portion 1, the guide recess 72 is slightly higher than the wheels 81 of the trolley 8. Thus, the wheels 81 can enter the guide mechanism 7 in a slightly tilted manner, thereby preventing the wheels 81 from getting stuck. Corresponding to the formation of the guide recesses 72, guide protrusions 71 are formed on upper portion of the guide recesses 72. The guide protrusions 71 enhance the strength of the guide mechanism 7. The structure of the guide mechanism 7 is not limited to the above-described embodiment; in other words, the guiding function can also be implemented by directly sticking projection stripes onto the inner walls of the recesses.

Furthermore, FIG. 5 schematically shows some of the devices carried on the trolley 8. These devices can be at least one of the endoscope driving apparatuses CV-260, CVL-260 and PSD-30 of Olympus Co., Ltd.

The following relates a description of the front side of the carrying portion 1.

As shown in FIGS. 1-3, the front side of the carrying portion 1 is provided with a wire arrangement box 5, a door 4 corresponding to the recessed portion 2 which has been described in detailed, and a pedal 9 that is operated by the doctor or nurse when he/she is operating the auxiliary equipment such as an aspirator.

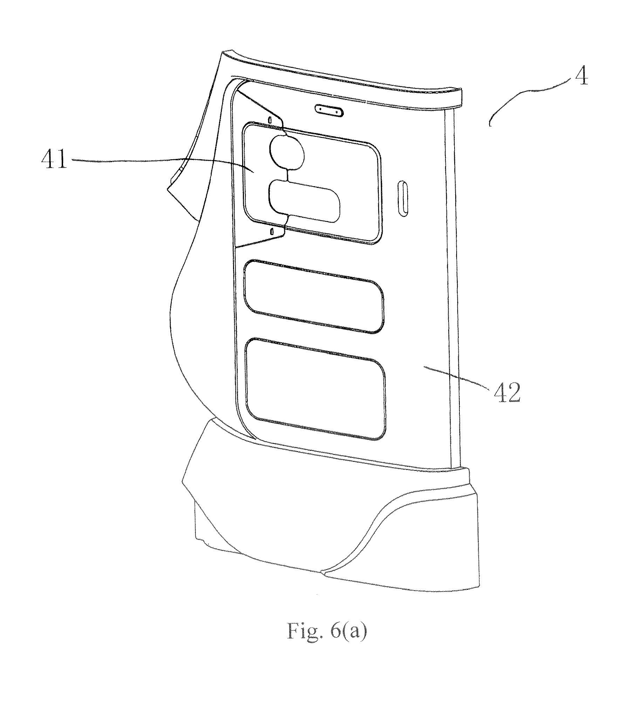

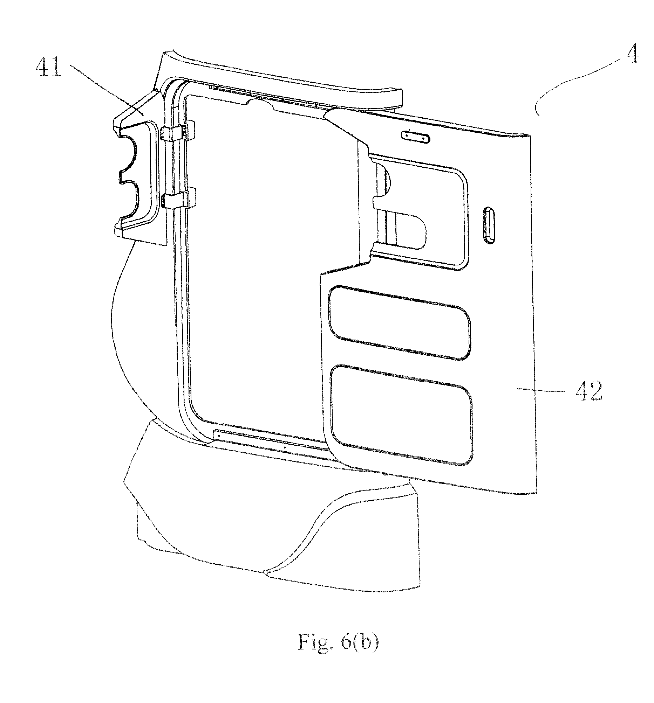

At a position on the front portion of the carrying portion 1 that is corresponding to the recessed portion 2, there is an opening which communicates with the recessed portion 2. The medical personnel can operate the medical equipment carried on the trolley 8 via the opening. A door 4 that can close the opening is provided at the opening and comprises a first door 41 that is relatively small and a second door 42 that is relatively big. As shown in FIGS. 6(a) and 6(b), the first door 41 is arranged on an upper left portion of the door 4, and is corresponding to the knobs of the frequently-used devices carried on the trolley, such as an endoscope image processing center and an endoscope cold light source, and is also corresponding to the frequently-used interfaces on the above devices, so that the medical personnel can conveniently carry out common operations of the devices and can easily insert the frequently-used wires into the interfaces by opening only the smaller first door 41. Further, the first door 41 is opened by means of rotation of a hinge, while the second door 42 is a slide door. The hinge of the first door 41 and the slide rail of the second door 42 can be selected from the hinges and the slide rails existing in the prior art. Furthermore, as shown in FIGS. 6(a) and 6(b), the door 4 is provided with a transparent window that is corresponding to the devices carried on the trolley 8.

It is also feasible to provide a lock mechanism between the first door 41 and the second door 42 to lock the smaller first door 41 on the second door 42. A lock mechanism can also be provided between the second door 42 and the carrying portion 1 so that the second door 42 can be fixed to the carrying portion 1.

Another technical solution of the present invention provides a door. In this technical solution, a cutout portion that allows wires of the medical equipment to pass through is formed on a part where the first door 41 and the second door 42 join each other. As shown in FIG. 6(b), in the present embodiment, a first cutout portion 41a is formed on the first door 41, and a second cutout portion 42a is formed on the second door 42. Thus, the first cutout portion 41 a and the second cutout portion 42a constitute a cutout portion for the wires of the medical equipment to pass through. However, the cutout portion is not limited to the above-described one; in other words, the cutout portion can be formed only on the first door 41 or the second door 42, which can achieve the same effect as the above cutout portion formed by two butted cutout portions. Owing to such a structure, it is possible for the medical personnel to directly insert wires such as the wires of the endoscope, especially the power supply plugs and light source cables, into the devices on the trolley, thereby further easing the uncomfortable feeling of the patient and making the entire operation area look tidier and cleaner.

The cutout portion can also be arranged on a part where the first door 41 and the second door 42 join each other, thereby forming a insert opening by the cutout portion between the first door 41 and the second door 42. Thus, even if the wires and the plugs, such as the plug of the endoscope, have been inserted into the trolley 8, the opening and closing of the first door 41 and/or the second door 42 will not be affected. In other words, even if the wires and the plugs, such as the plug of the endoscope, have been inserted into the trolley 8, the operator can still open the first door 41 to operate the frequently-used buttons, and/or open the second door 42 to operate the buttons that are not often used.

Next, the wire arrangement box 5 will be described. The wire arrangement box 5 is disposed within the carrying portion 1. The wire arrangement box 5 communicates with the recessed portion 2 so that the wires of the various devices on the trolley 8 (including the wires of the endoscope, such as the power supply wire, the signal line and the suction duct) can be connected to the wire arrangement box 5 or the display apparatus. It is feasible to provide within the carrying portion 1 a passage that is used specially for wiring. Alternatively, it is feasible to preset various connectors directly on the carrying portion 1 so that the power supply and the signal transmission between the devices can be achieved only by connecting the wires (especially the power supply wire and the signal line, also including the line of the aspirator of the endoscope that is connected to the endoscope driving apparatus) on the trolley to the corresponding standard connectors. Furthermore, it is also feasible not to provide any device within the carrying portion 1, i.e., keeping the carrying portion empty and using a wire support to support the wires. Thus the wires can be more directly routed. According to the present invention, the various wires are arranged within the carrying portion, so the unneeded wires won't be seen by the medical personnel or the patient and the room for diagnosis and treatment can be kept clean and tidy.

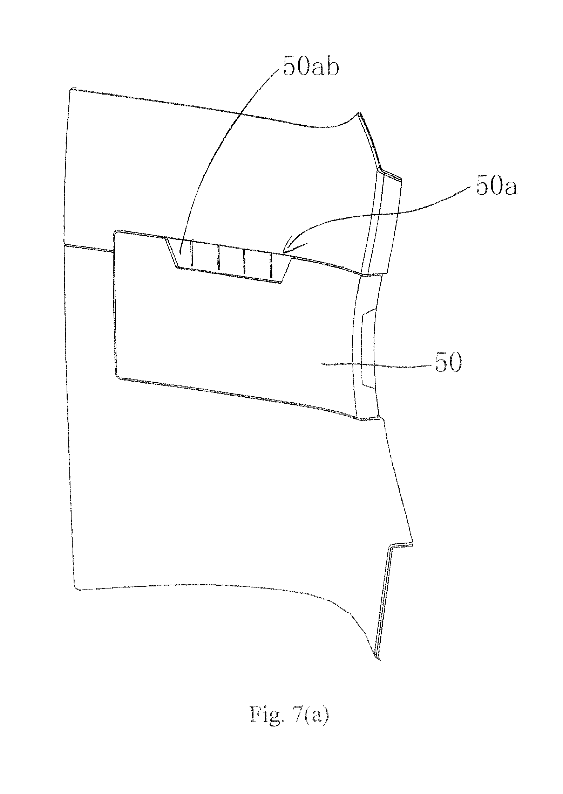

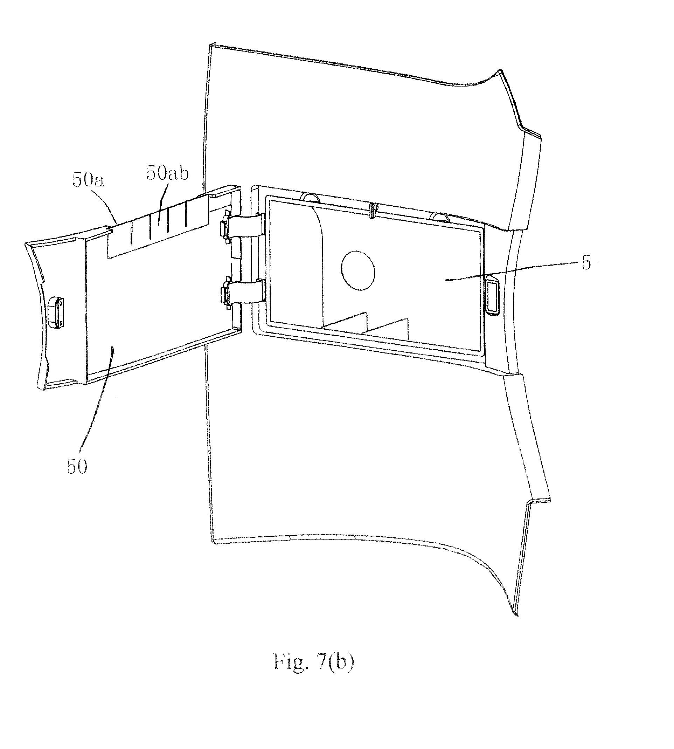

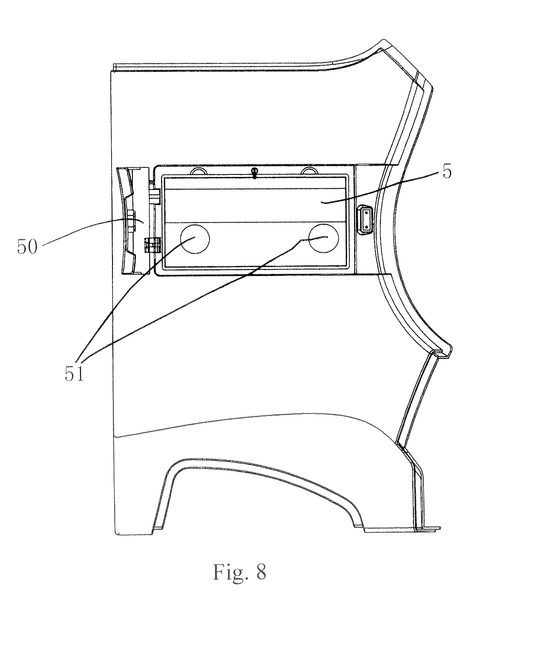

As shown in FIGS. 7(a), 7(b) and 8, a rear wall of the wire arrangement box 5 is provided with two lead openings. One of the lead openings is used for leading out the suction duct while the other one is used for leading out the wires of the devices. Further, a lower portion of the wire arrangement box 5 is provided with wire-receiving recessed portions which are independent of one another. Each wire-receiving recessed portion corresponds to a lead opening such that the wires extracted from each lead opening are stored in the corresponding wire-receiving recessed portion. Thus, there is no need to frequently extract the wires from the lead openings, thereby reducing labor intensity of the medical personnel.

The most distinctive characteristic of the wire arrangement box 5 lies in the door 50 for the wire arrangement box. The door 50 is mounted to the wire arrangement box 5 by means of a hinge. The hinge can be any hinge that is on the market. Furthermore, the door 50 for the wire arrangement box is provided with a lock means, e.g. a lock, for locking the door 50 on the wire arrangement box 5. An upper portion of the door 50 for the wire arrangement box 5 is provided with a damping mechanism 50a for releasing and retracting the wires. The damping mechanism 50a applies a damping force to the wires that are released from the wire arrangement box 5 so that the wires won't be released excessively or the wires won't retract automatically due to gravity, thereby providing convenience to the operation of the medical personnel.

As shown in FIGS. 7(a) and 7(b), in the present embodiment, the damping mechanism 50a for releasing and retracting the wires is formed by a single rubber sheet (usually made of silica gel). The rubber sheet is processed to have a plurality of incomplete notches in a longitudinal direction, thereby forming a plurality of rubber sheets 50ab. Thus, the wires enter the notches between the rubber sheets 50ab which apply a damping force to the wires. The damping mechanism 50a in the present embodiment only serves as an example; that is, the present invention is not limited to this, and the rubber sheets can be replaced by damping roller wheels or gripping mechanism having a damping function.

Let's return to FIG. 1 to give a description of a pedal 9 that is arranged at the lower portion of the carrying portion 1.

As shown in FIG. 1, the pedal 9 can be stored in the carrying portion 1. Different from the situation that in the past the pedal was placed directly on the ground, the structure of the present invention makes it possible to store the pedal 9 in the carrying portion 1 when the pedal 9 is not used. Thus, when the pedal 9 is not used, the room for diagnosis and treatment looks clean and tidy, and the medical personnel or the patients won't be tripped up by the pedal 9. Further, the pedal slides out via a slide rail in the present embodiment, but the present invention is not limited to this. It is also feasible to provide a guide groove on the carrying portion 1 to guide the pedal 9. Another possible design is to provide a pop-up mechanism on the carrying portion 1 such that the pedal 9 pops up when being pressed and the pedal 9 can be fixed within the carrying portion I by means of an engagement mechanism after the pedal 9 is returned to the carrying portion 1.

Furthermore, in the present embodiment the pedal and the carrying portion are connected to each other by cable, but it is also feasible to provide a wireless transceiver on the pedal 9 and on the carrying portion 1 respectively to effect wireless signal transmission between the pedal and the carrying portion 1.

Next, a joining mechanism for connecting and engaging the carrying portion I with the operation portion 100 is to be described.

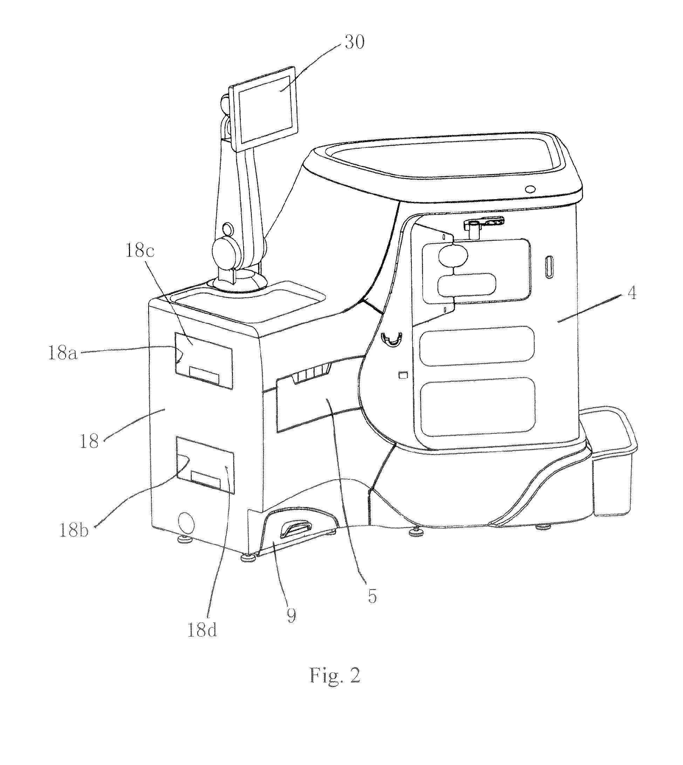

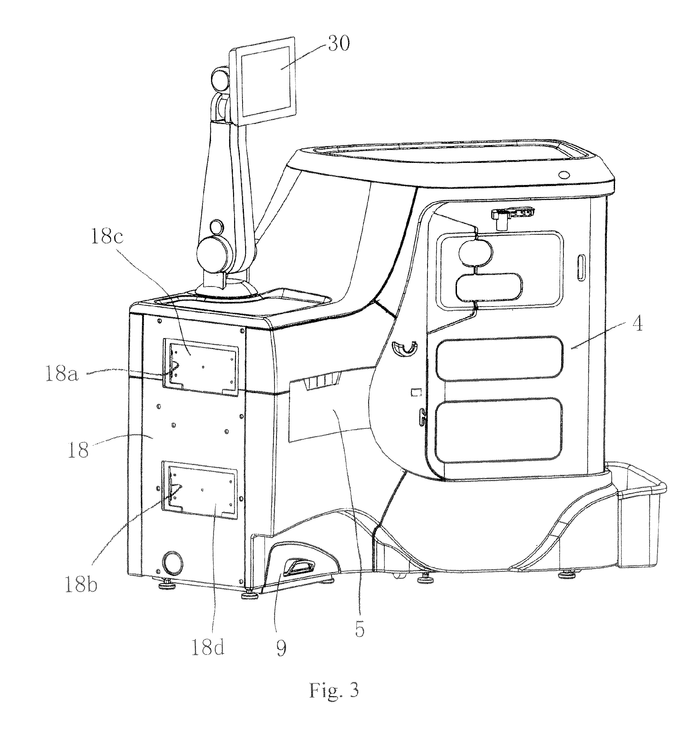

The joining mechanism of the present invention connects the carrying portion 1 to the operation portion 100 by means of a recess-protrusion engagement. As shown in Figs. 2 and 3, the carrying portion I has a planar end portion 18. A joining opening 18a is provided on an upper part of the end portion 18 and another joining opening 18b is provided on a lower part of the end portion 18. Slide rails can be provided in the joining openings 18a and 18b so that drawers which can accommodate articles used by the medical personnel can be inserted into the joining openings 18a and 18b when the carrying portion 1 is used alone. When the drawers are not used, the medical personnel can detach them from the carrying portion 1 and mount joining protrusions 18c and 18d respectively to the joining openings 18a and 18b. As the operation portion 100 is pre-provided with an end face (not shown) which fits with the end face 18 and with joining recesses which engage with the joining protrusions 18c and 18d, the connection between the operation portion 100 and the carrying portion 1 can be achieved as long as the joining protrusions 18c and 18d engage with the joining recesses on the operation portion 100. The cable connection between the operation portion 100 and the carrying portion 1 can be effected by providing between the operation portion 100 and the carrying portion 1 an insert hole that can be covered by a cover plate. As to the wire connection, a person skilled in the art can make a design based on the actual need, so it is unnecessary to go into details.

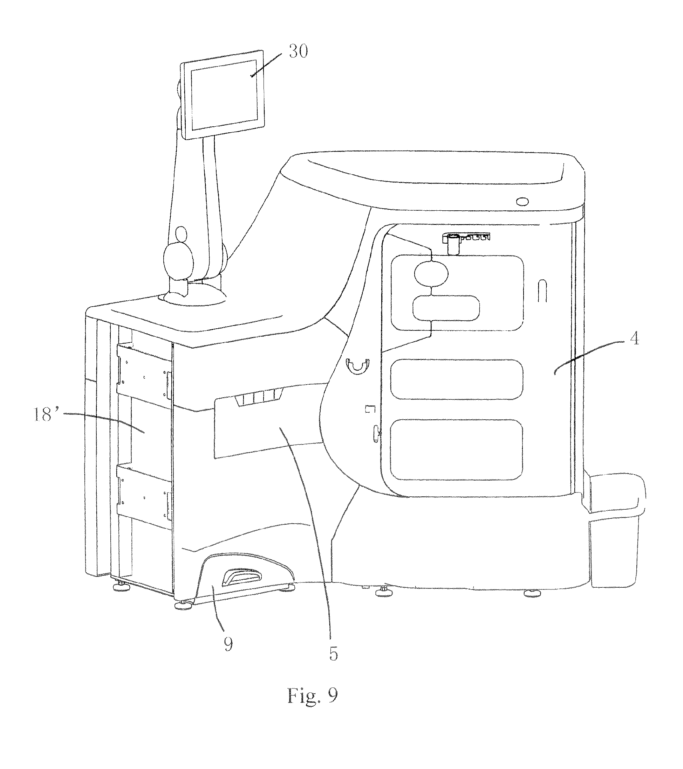

Further, regarding the joining mechanism, as shown in FIG. 9, it is also feasible to provide the joining recesses on an end face 18' of a carrying portion 1' in another embodiment. The operation portion 100 is provided with protrusions corresponding to the end face 18'. As shown in FIG. 9, the joining recesses on the end face 18' have the same width in the longitudinal direction, so it is possible to provide joining plates in the joining recesses and use the engagement between the joining plates and the joining recesses on the protrusions on the operation portion 100 to restrict a displacement between the carrying portion 18' and the operation portion 100 in an up-and-down direction and use the engagement between the joining recesses on the end face 18' and the corresponding protrusions on a support 100 to restrict a displacement between the carrying portion 18' and the operation portion 100 in a forward-and-backward direction. Thus, by means of the above-described structure, it is possible to achieve a more secure connection and fixation between the carrying portion 18' and the operation portion 100.

Regarding the connection and fixation between the carrying portion and the operation portion, it is also feasible to further provide an engagement mechanism or a fixing mechanism. For example, it is feasible to provide click-type clips that can fix the carrying portion to the operation portion. There is no special limitation on the form of the engagement mechanism or the fixing mechanism, as long as the engagement mechanism or the fixing mechanism can restrict the relative displacement between the carrying portion and the operation portion. Alternatively, the joining surfaces can be flat surfaces that are fixed to each other by bolts.

Furthermore, if necessary, it is also feasible to provide on the carrying portion 1 the devices such as a white balance cap for adjusting the development effect of the endoscope and heat dissipating grids for allowing the air to circulate. Furthermore, the rear portion of the carrying portion 1 is provided with a plurality of suction bottles which recover liquid aspirated from the human body. The carrying portion 1 can be further provided with water-feeding bottles for containing lavage solution e.g. water that is injected into the human body. An area of the carrying portion is used for holding articles used by the medical personnel, and the outer edge of the area is provided with a protrusion which prevents the medicines or the medical instruments from falling off the carrying portion.

The carrying portion I is further provided with a sub-monitor 30 that is used by the nurses and other paramedics for observation. Although the carrying portion 1 in the present embodiment has the sub-monitor 30, it is also possible to provide only a sub-monitor mount member on the workstation and let the medical personnel decide whether to mount a sub-monitor or not or when to mount a sub-monitor.

The bottom of the carrying portion 1 is provided with a plurality of adjusting feet whose lengths can be adjusted. By adjusting the lengths of the adjusting feet, the user can adjust the overall height of the carrying portion 1. Or, the user can adjust the lengths of some of the adjusting feet to keep a balance of the entire carrying portion to thereby prevent the carrying portion from wobbling due to the unevenness of the bottom.

It is also feasible to provide on the carrying portion a shortcut key. The shortcut key has a large surface area and is arranged in a position that can be easily pressed by the doctor. By pressing the shortcut key, the doctor can control the automatic capturing of screen shots of the computer or call the nurses.

Further, the endoscope system carrying portion 1 and the operation portion 100 of the medical endoscope system workstation described in the embodiments in this description can be detached from each other. However, it is self-evident that the endoscope system carrying portion 1 and the operation portion 100 in the above embodiments can also be formed as an integral and an undetachable one.

For example, the endoscope system carrying portion can be formed into an elongated structure (the elongated structure means that the length of the structure in one direction is longer than the length of the structure in another direction perpendicular to said direction), and the trolley storage portion is arranged near an end portion of the endoscope system carrying portion, and a main monitor bracket is provided near another end portion of the endoscope system carrying portion. The structure of this main monitor bracket is similar to that of the above-described main monitor bracket on the operation portion 100; that is to say, this main monitor bracket comprises a main strut rod and a universal adjusting mechanism that is disposed on the upper end of the main strut rod and consists of a plurality of sub strut rods, and the universal adjusting mechanism is provided with a damper for retaining the universal adjusting mechanism in an adjusted position, and the endoscope system carrying portion is provided with a weight for preventing the endoscope system carrying portion from overturning.

Thus, it is possible to use an integral endoscope system carrying portion to replace the above-described endoscope system carrying portion 1 to function as an endoscope system workstation. This structure simplifies the overall structure of the endoscope system carrying portion (or the workstation) and makes it easier to place the carrying portion at the scene of the medical institution.

With this structure, the components and the structures of the other parts of the carrying portion can be the same as those of the corresponding pars of the carrying portion in the above embodiment 1.

The trolley storage portion is not limited to the above recessed portion 2. For example, the trolley storage portion can take the form of the following variant.

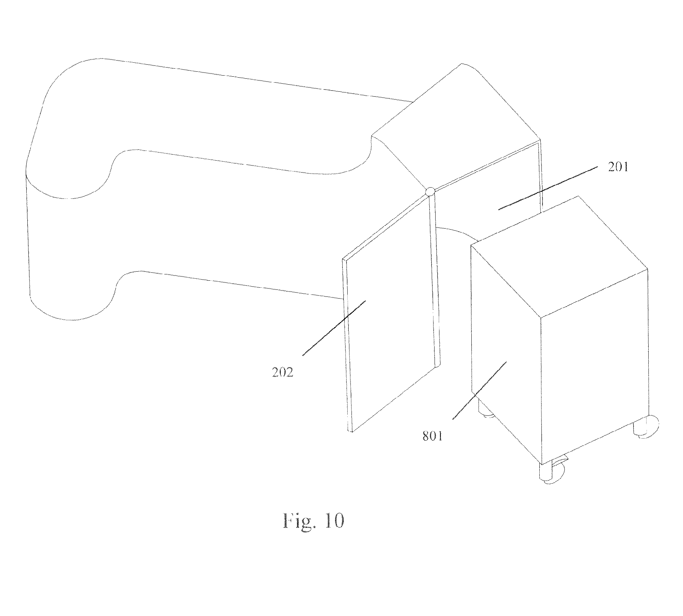

For example, as shown in FIG. 10, a recessed portion 201 which serves as the trolley storage portion opens on one side of the carrying portion. As shown in FIG. 10, a door 202 for closing the opening can be provided at the opening of the recessed portion 201. Thus, after a trolley 801 is pushed into the recessed portion 201, by shutting the door 202, the user can get a clean appearance of the entire carrying portion (or the workstation).

Undoubtedly, it is also feasible to omit the door 202. In this situation, after the trolley is pushed into the recessed portion 201, a rear portion of the trolley covers the opening of the recessed portion 201.

In the above variant as shown in FIG. 10, the opening of the recessed portion, 201 opens toward one side of the carrying portion, but the present invention is not limited to this. For example, the opening of the recessed portion can open toward the front of the carrying portion. In this situation, one can choose to have a door or not to have a door. By means of the structure of this variant, one can obtain an effect that is the same as that produced by the above variant as shown in FIG. 10. Furthermore, in the above embodiments and variants, after the trolley is pushed into the recessed portion, the rear side of the trolley is exposed. However, the rear side of the trolley can have a rear panel that can be made of a material that is the same as the material of the housing of the carrying portion. Thus, after the trolley is pushed into the recessed portion, the surface of the entire carrying portion is an integral whole, which helps to improve the appearance of the carrying portion.

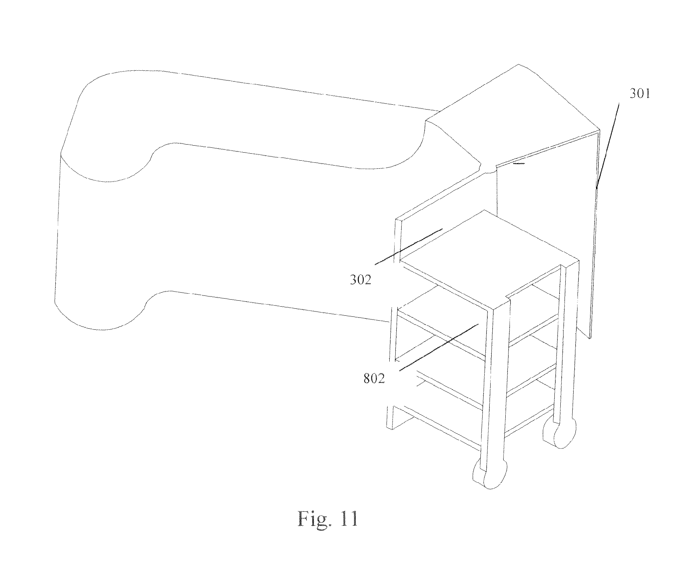

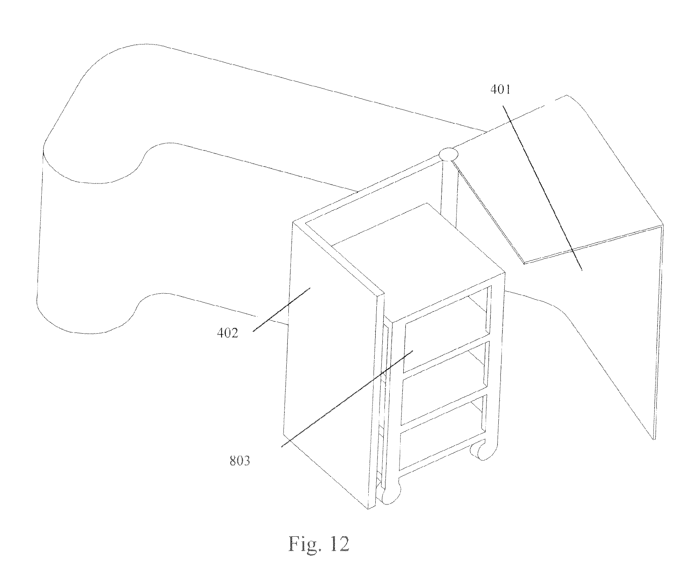

As shown in FIGS. 11 and 12, it is also feasible to provide a door 302 or 402 at the opening of the recessed portion. One side of the door 302 or 402 is rotatably mounted to the carrying portion by a hinge, and a trolley 802 or 803 is fixed to the door 302 or 402. The difference between FIG. 11 and FIG. 12 is that the recessed portion 301 in FIG. 11 opens only toward one direction of the carrying portion (i.e., toward one side of the carrying portion; however, the recessed portion 301 can also open toward the front or the back of the carrying portion), whereas the recessed portion 401 in FIG. 12 opens toward one side and the front of the carrying portion (the direction in which the recessed portion opens is not limited to the direction specified here; that is, the recessed portion can open toward the side and the back of the carrying portion).

As shown in FIG. 11, the door 302 is fitted to one side of the recessed portion 301 by a hinge. Further, the trolley 802 is integrally mounted to the door 302. Thus, while the door 302 is being closed, the trolley 802 enters the recessed portion 301. Such a design provides a compact carrying portion that is easy to operate.

As shown in FIG. 12, the recessed portion 401 opens toward the front and the side of the carrying portion, and the door 402 is fitted to one side of the opening of the recessed portion 401 by a hinge. When the door 402 is being closed, it closes the opening of the recessed portion 401 which opens toward both the front and the side of the carrying portion. Further, in FIG. 12, two sides on the upper edge of the recessed portion 401 are substantially perpendicular to each other, and the shape of the door 402 matches with the shape of the opening of the recessed portion 401. Thus, by means of the engagement of the door 402 and the opening of the recessed portion 401, the opening of the recessed portion 401 is closed by the door 402, thereby improving the overall appearance of the carrying portion.

As shown in FIG. 12, the trolley 803 is integrally mounted to one side of the door 402 that faces the recessed portion 401. Thus, like the structure as shown in FIG. 11, the structure shown in FIG. 12 can also provide a compact carrying portion that is easy to operate.

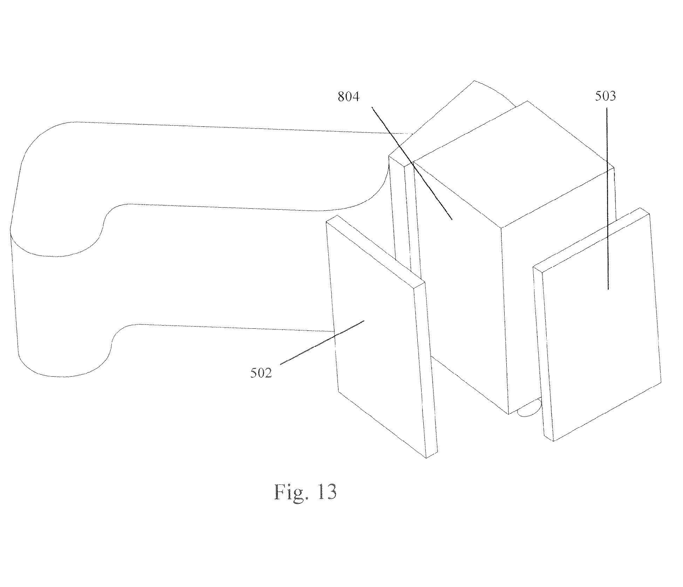

The trolley storage portion of the carrying portion of the present invention can also have a structure as shown in FIG. 13. FIG. 13 shows a structure that a trolley 804 is sealed in the carrying portion. For the sake of conciseness and clarity, FIG. 13 only shows a front panel 502 and a side panel 503 of the trolley storage portion and omits an upper panel and a rear panel of the trolley storage portion. After all the panels are mounted to the carrying portion, a trolley storage portion (the trolley storage space) for accommodating the trolley 804 is formed. The structure shown in FIG. 13 is mainly used in the situation where the trolley needs to be sealed in the trolley storage portion of the carrying portion. Although the trolley 804 in FIG. 13 has wheels, it is also feasible for the trolley 804 to have no wheels.

The structures shown in FIGS. 11-13 are especially effective in the situation where the trolley does not need to be replaced frequently.

The above description of the variants only explains the structure of the trolley storage portion and the structure of the trolley. The structures and functions of other parts of the carrying portions in the above-described variants are the same as those in the above embodiment.

Furthermore, on the basis of the above embodiment, the following improvement can be made to the present invention.

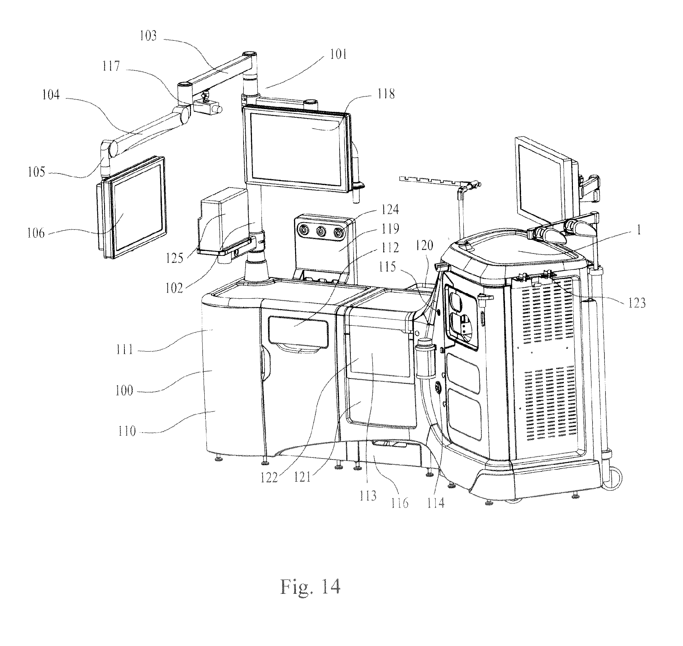

As shown in FIG. 14, the operation portion 100 is provided with a main monitor bracket 101. The main monitor bracket 101 comprises a main strut rod 102 and a universal adjusting mechanism that is disposed on the upper end of the main strut rod 102 and consists of a plurality of sub strut rods 103-105. The universal adjusting mechanism is provided with a damper for retaining the universal adjusting mechanism in an adjusted position. The main strut rod 102 is provided on a rear end portion on an upper surface of the operation portion 100 in a forward/backward direction, and the sub strut rods 103-105 extend in the forward/backward direction in a manner of transversely striding over the operation portion 100. In this way, the weight of the main strut rod 102 can improve the stability of the entire operation portion. Furthermore, that the main strut rod 102 is arranged on the rear end portion of the operation portion 100 facilitates the installation and the removal of the main strut rod 102 and increases the available space on the front part of the operating desk.

Further, as shown in FIG. 14, the number of the main monitor 106 changes from two to one, because the doctor is apt to have visual fatigue when the two main monitors are too close to each other. Moreover, the radian at a front end portion 110 of the operation portion becomes small, which reduces the area covered by the operation portion and increases the standing space of the anesthetist. The arc-shaped portion 110 has a storage space with a door 111. The weight that was originally provided at the arc-shaped portion 110 is canceled, which reduces the weight of the whole system and increases the space for storing the consumable material.

As to the improvement to the entire rear portion of the system (drawing omitted), it is feasible to arrange the wiring groove, (the wiring groove is such a space that leads the wires to be connected with other devices from the front portion to the rear portion and accommodates the wires) which was originally disposed inside the system, at the bottom of the back side of the system. Such an arrangement facilitates the wiring operation and the examination and repair of cable failures. To be specific, before the above improvement to the rear portion of the system, when the wiring operation needs to be carried out, it is necessary to remove the backplate and round other devices inside the system to operate the wiring in the wiring groove, which is quite inconvenient; however, after the wiring groove is arranged at the bottom of the back side of the system, the wiring groove can be presented directly in front of the user only by removing the backplate, so that the examination and repair of failures become more convenient and faster. Furthermore, the backplate was originally installed by means of screws while it is now installed and removed by means of clicks, and the central portion of the backplate is provided with a pull-tab assembly which comprises a pull-tab groove provided in an inward recess on the backplate and a pull-tab hinged to the pull-tab groove at one end. When the system works normally, the pull-tab is stored in the pull-tab groove. When the backplate needs to be removed, the pull-tab is pulled and the clicks on the backplate are disengaged automatically; or the user pulls the backplate by pulling the pull-tab along a direction in which the clicks of the backplate are disengaged, such that the backplate can be easily removed. Thus, no special tools are needed, and the user only needs to manually disengage the clicks and opens the backplate to operate the devices inside the system.

As shown in FIG. 14, the front portion of the operation portion 100 is further provided with a drawer 112 which holds consumable materials that are commonly used in diagnosis and treatment. The front portion (a position corresponding to the wire arrangement box 5 in FIG. 1; and the wire arrangement box 5 is canceled in FIG. 14) of the carrying portion 1 is provided with a storage space for accommodating medical devices such as ESG-100, AFU-100 and their lead wires. The storage space has an opening at the front portion of the endoscope system carrying portion 1, and a door 113 (made of transparent material) that can be slidably opened and closed is provided at the opening. To be specific, as shown in FIG. 14, at the front portion of the carrying portion 1, an accommodating groove 121 of the door of the storage space is provided under the opening of the storage space. Here, both ends on the back side of the door 113 of the storage space are slidably mounted to the opening in a vertical direction by means of a vertically-disposed slide guide rail assembly (not shown). When the storage space needs to be opened, the door 113 slides downward by means of the slide guide rail assembly, and gradually slides into the accommodating groove 121. When the opening needs to be closed, the door 113 moves upward along the slide guide rail and closes the storage space.

At the front portion of the carrying portion 1, a suction line 114 and a temporary storing area 115 for storing a suction duct are provided near the transparent door 113 (on the right side of the door 113 in FIG. 14). The suction line 114 is a dedicated passage, which prevents the suction duct from polluting other cables.

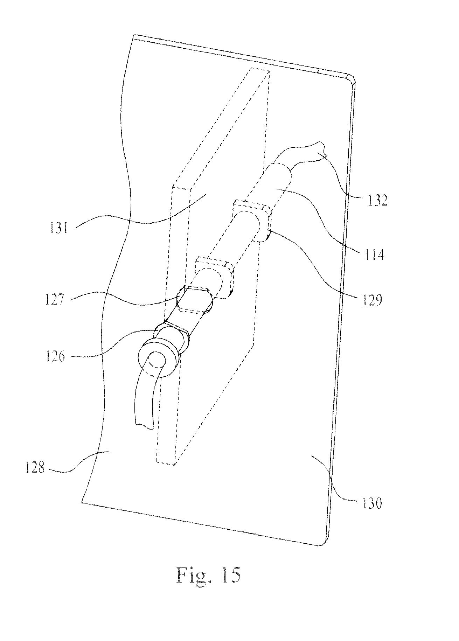

FIG. 15 is a simplified schematic view showing how the suction line 114 is mounted to the endoscope system carrying portion 1 (hereinafter referred to as the carrying portion 1). In FIG. 15, the reference number 130 indicates a front plate of the carrying portion 1 and the reference number 131 indicates a component fixed in a certain position inside the carrying portion 1. For the sake of convenience, a description as to how the suction line 114 is mounted to the carrying portion 1 will be given, with other members (e.g. the bottle AFU-100 and the water feeding bottle) on and inside the front plate 130 being omitted. As shown in FIG. 15, the suction line 114 is a passage that penetrates from the front side of the carrying portion 1 to the rear side of the carrying portion 1, and a suction duct 132 can be inserted into the interior of the suction line 114 and guided from the front side of the carrying portion 1 to a suction bottle on the rear side of the carrying portion 1. The suction line 114 and the carrying portion 1 are connected to each other in the following manner: the front plate 130 and the backplate of the carrying portion 1 are each provided with an opening, and the interior of the carrying portion 1 is provided with a space that the suction line 114 penetrates. As shown in FIG. 15, the space contains two suction-line fixing frames 129, and one end of each fixing frame 129 is fixed to the component 131 that is fixed in a certain position inside the carrying portion 1, and the central portion of each fixing frame 129 is a hole that the suction line 114 can pass through, such that the suction line is fixed by means of the openings on the front plate 130 and the backplate and the holes at the central portions of the fixing frames 129. In a position on the suction line 114 that is slightly behind the front port of the suction line 114, at least a pair of engaging projections 126 is provided in at least a group of positions that are radially opposite to each other; and in the space for accommodating the suction line 114, there is an engaging portion 127 that is capable of engaging with the engaging projections 126 to thereby prevent the suction line 114 from being pulled out. To be specific, as shown in FIG. 15, the section of the engaging projection 126 has an elongated circular shape (i.e., a shape that is formed by joining two semicircles respectively on the two shorter sides of a rectangle), and the engaging portion 127 is an elongate hole that is provided on the front plate 130 and has a shape and a size substantially corresponding to the shape of the section of the engaging projection 126. The engagement of the engaging projections 126 and the engaging portion 127 is achieved by rotating the suction line 114 after the suction line 114 is inserted into the space for accommodating the suction line 114. The following is a detailed description of the process of attaching and removing the suction line 114. When the engaging projections 126 are rotated to a position that completely coincides with the elongate hole and are inserted into the elongate hole, the user only needs to slightly rotate the suction line 114 with respect to the elongate hole to make the longer sides of the engaging projections 126 and the longer sides of the elongate hole shifted from each other by a certain angle, restriction of the suction line 114 in the forward/backward direction can be achieved; on the contrary, when the suction line 114 needs to be removed, the user rotates the suction line 114 to make the longer sides of the engaging projections 126 and the longer sides of the elongate hole coincide with each other again and pulls out the suction line 114. Such a design makes it easy to remove the suction line 114 (a knob structure is employed, so the suction line can be readily removed merely by turning and pulling the suction line, without using any special tools). The structures and shapes of the engaging projections 126 and the engaging portion 127 of the present invention are not limited to those described above; that is, other types of engaging structure are also acceptable.

The carrying portion 1 is further provided with a temporary storing area 115 for storing the suction duct. The temporary storing area 115 is a rubber cap having a bottom. The rubber cap has a hole (not shown) that opens toward the front of the endoscope system carrying portion 1 and engages with the head of the suction duct. While the endoscope is being changed, the temporary storing area 115 provides a place for temporarily storing the suction duct. Moreover, the temporary storing area 115 can be removed from the carrying portion to be cleaned or replaced independently.

As shown in FIG. 14, a further improvement involves canceling the sub monitor 30 shown in FIG. 1, and the function of the sub monitor 30 can be performed by the monitors on the trolley. Furthermore, a drawer 116 is provided in a position that is corresponding to the pedal 9 at the lower portion of the endoscope system carrying portion 1, so the pedal 9 can be stored in the drawer 116 and thus the cleaning of the floor is made easier. After the pedal 9 is taken out from the drawer 116, the drawer can be closed (the wires can be extracted via an opening on the upper portion of the drawer). The sub strut rod 103 is provided with a camera 117 which can record the operations at the scene of diagnosis and treatment, and the videos can be used on academic conferences. If necessary, the main strut rod 102 can be further provided with a monitor 118 that can be rotated around a vertical direction. The operation portion 100 is provided with a gas integration device 119 and the carrying portion 1 is provided with an ET support 120. The gas integration device 119 integrates the gas supply devices so that the nurses can operate the gas supply devices without making any movements or only by making few movements. The gas integration device 119 is a plate structure that is disposed perpendicular to the operation portion 100 and is provided with a plurality of connector components 124 that communicate with medical gas apparatuses (such as oxygen cylinders). The connector components 124 can be designed differently according to different types of gas apparatus that are actually used so as to match with the gas supply connectors of the gas apparatus. The ET support 120 is used for hanging an operation component during the diagnosis and treatment. Further, if necessary, it is feasible to mount a patient monitor 125 (the patient monitor 125 can be rotated around the main strut rod 102 by 270°; as shown in FIG. 14, the patient monitor 125 is rotated to a position that is perpendicular to the panel of the monitor 118) to the lower portion of the main monitor bracket 101.

At the right tip end of the carrying portion, there is a positioning bracket 123 for temporarily storing the operation portion of the endoscope system.

To sum up, it should be understood that one skilled in the art can carry out various changes and modifications within the range of the concept of the present invention and without departing from the protection scope of the present invention.

## Claims

1. An endoscope system carrying portion, wherein the endoscope system carrying portion (1) is provided with a trolley storage portion (2) for storing a trolley (8) which carries medical equipment including at least an endoscope driving apparatus (4).

2. The endoscope system carrying portion according to claim 1, wherein the trolley storage portion (2) is a recessed portion (2) that is provided on the endoscope system carrying portion (1) and opens rearward, and
an opening that communicates with the trolley storage portion (2) is provided at a position on a front portion of the endoscope system carrying portion (1) that is corresponding to the trolley storage portion (2), and operations of the medical equipment carried on the trolley (8) can be carried out via the opening, and a door (4) capable of closing the opening is provided at the opening.

3. The endoscope system carrying portion according to claim 2, wherein the door (4) consists of a first door (41) that is arranged in a position corresponding to frequently-used buttons and/or interfaces of the medical equipment carried on the trolley (8), and a second door (42).

4. The endoscope system carrying portion according to claim 3, wherein the first door (41) is smaller than the second door (42).

5. The endoscope system carrying portion according to any one of claims 1-4, wherein the endoscope system carrying portion (1) is further provided with a storage space which has an opening on the front portion of the endoscope system carrying portion (1), and a door (113) for the storage space that can be slidably opened and closed is provided at the opening.

6. The endoscope system carrying portion according to any one of claims 1-4, wherein a temporary storing area (115) for storing a suction duct, which area is a rubber cap having a bottom, is provided on the front portion of the endoscope system carrying portion (1), and the rubber cap has a hole that opens toward the front of the endoscope system carrying portion (1) and for engaging with a head of the suction duct.

7. The endoscope system carrying portion according to any one of claims 1-4, wherein the endoscope system carrying portion (1) further comprises a suction line (114) for a suction duct to pass through; an interior of the endoscope system carrying portion (1) is provided with a space through which the suction line (11) passes from a front side of the endoscope system carrying portion (1) to a rear side of the endoscope system carrying portion (1); in a position on the suction line (114) that is slightly behind a front port of the suction line (14), at least a pair of engaging protrusions (126) is provided in at least a group of radially opposite positions, and in the space for accommodating the suction line (114), there is also an engaging portion (127) capable of engaging with the engaging protrusions to prevent the suction line (114) from being pulled out.

8. The endoscope system carrying portion according to any one of claims 1-4, wherein the endoscope system carrying portion (1) is further provided with a wire arrangement box (5) whose rear portion communicates with the trolley storage portion (2); the wire arrangement box (5) has an opening on the front portion of the endoscope system carrying portion (1); a door (50) for the wire arrangement box which has a damping mechanism (50a) for releasing and retracting wires is provided at the opening of the wire arrangement box (5).

9. The endoscope system carrying portion according to claim 8, wherein the damping mechanism (50a) for releasing and retracting wires is arranged on an upper portion of the door (50) for the wire arrangement box (5) and consists of a plurality of sheet-like rubber bodies (50ab) which are arranged side by side on the door (50) for the wire arrangement box; and a damping force is applied to wires that are released and retracted via the door (50) for the wire arrangement box by means of the respective sheet-like rubber bodies (50ab).

10. The endoscope system carrying portion according to any one of claims 1-9, wherein a trolley guide mechanism (7) for guiding the trolley (8) is provided within the trolley storage portion (2); and the trolley guide mechanism (7) is disposed at a bottom of the trolley storage portion (2) and has a shape that matches with the shape of the wheels of the trolley (8).

11. The endoscope system carrying portion according to any one of claims 1-9, further comprising at least one of the following devices:
a sub monitor (30) mounted to an upper portion of the endoscope system carrying portion (1);
a pedal (9) mounted to a lower portion of the endoscope system carrying portion (1);
a white balance cap provided on a side portion of the endoscope system carrying portion (1);
a suction bottle provided on a rear portion of the endoscope system carrying portion (1); and
adjusting feet provided on the bottom of the endoscope system carrying portion (1).

12. The endoscope system carrying portion according to any one of claims 1-11, wherein the endoscope system carrying portion (1) is further provided with a flat first end face (18) having at least one joining opening (18a, 18b); and a drawer or cover plate is provided at the joining opening (18a, 18b).

13. The endoscope system carrying portion according to any one of claims 1-11, wherein the endoscope system carrying portion is an elongated structure; the trolley storage portion is provided near an end portion of the endoscope system carrying portion; and a main monitor bracket for supporting a main monitor is provided near the other end portion of the endoscope system carrying portion.

14. The endoscope system carrying portion according to claim 13. wherein the main monitor bracket comprises a main strut rod and a universal adjusting mechanism that is disposed on an upper end of the main strut rod and consists of a plurality of sub strut rods; and the universal adjusting mechanism is provided with a damper for retaining the universal adjusting mechanism in an adjusted position, and
the main strut rod is provided at an end portion of the endoscope system carrying portion in a forward/backward direction, and the sub strut rods extend in the forward/backward direction in a manner of transversely striding over the endoscope system carrying portion (1).

15. The endoscope system carrying portion according to claim 13, wherein the main monitor bracket comprises a main strut rod and a universal adjusting mechanism that is disposed on an upper end of the main strut rod and consists of a plurality of sub strut rods; the universal adjusting mechanism is provided with a damper for retaining the universal adjusting mechanism in an adjusted position; and a weight for preventing the endoscope system carrying portion (1) from overturning is provided on the endoscope system carrying portion.

16. The endoscope system carrying portion according to claim 1, wherein the trolley storage portion (2) is one of the following structures:
a recessed portion that is provided on the endoscope system carrying portion (1) and opens forward;
a recessed portion that is provided on the endoscope system carrying portion (1) and opens sideward; and
a trolley storage space that is provided in the endoscope system carrying portion (1).

17. The endoscope system carrying portion according to claim 1, wherein the trolley storage portion (2) is a space enclosed by a recessed portion formed on the endoscope system carrying portion (1) and a door for closing the recessed portion; and the trolley is fixed to the door and can be stored within the trolley storage portion (2) as the door rotates.

18. An endoscope system workstation, comprising an operation portion (100) and the endoscope system carrying portion (1) according to any one of claims 1-11, which can be joined together by a joining mechanism.

19. An endoscope system workstation, comprising an operation portion (100) and the endoscope system carrying portion (1) according to claim 12,
wherein joining halves (18c, 18d) of the carrying portion, which project from the endoscope system carrying portion (1) or are recessed into the joining openings 18a, 18b), are provided at the joining openings (18a, 18b); and the operation portion (100) has a second end face that fits with the first end face (18) of the endoscope system carrying portion (1), and joining halves of the operation portion which fit with the joining halves (18c, 18d) of the carrying portion are provided on the second end face.

20. The endoscope system workstation according to claim 19, wherein a main monitor bracket (101), which comprises a main strut rod (102) and a universal adjusting mechanism that is disposed on an upper end of the main strut rod (102) and consists of a plurality of sub strut rods (103∼105), is provided on a middle portion of the operation portion (100); the universal adjusting mechanism has a damper for retaining the universal adjusting mechanism in an adjusted position; and a lower portion of the operation portion (100) is provided with a weight for preventing the operation portion (100) from overturning.

21. The endoscope system workstation according to claim 19, wherein the operation portion (100) is provided with a main monitor bracket (101) which comprises a main strut rod (102) and a universal adjusting mechanism that is disposed on an upper end of the main strut rod (102) and consists of a plurality of sub strut rods (103∼105); the universal adjusting mechanism has a damper for retaining the universal adjusting mechanism in an adjusted position; and the main strut rod (102) is arranged on an end portion of the operation portion (100) in a forward/backward direction, and the sub strut rods (103∼105) extend in the forward/backward direction in a manner of transversely striding over the operation portion (100).
